# FASCICULE DE BREVET EUROPEEN

(11) **EP 3 313 298 B1**
(45) Date de publication et mention de la délivrance du brevet: **01.05.2019**
(21) Numéro de dépôt: 16747810.6
(22) Date de dépôt: 23.06.2016
(51) Int. Cl.: A61B 17/15, A61B 17/17

(54) **ENSEMBLE D'INSTRUMENTS JETABLES POUR UNE OPERATION CHIRURGICALE D'UN PATIENT, ET PROCÉDÉ DE FABRICATION DUDIT ENSEMBLE**
SATZ AUS EINWEGINSTRUMENTEN FÜR EINEN CHIRURGISCHEN EINGRIFF AN EINEM PATIENTEN UND VERFAHREN ZUR HERSTELLUNG SOLCH EINES SATZES
SET OF DISPOSABLE INSTRUMENTS FOR A SURGICAL OPERATION ON A PATIENT, AND METHOD FOR THE PRODUCTION OF SAID SET

(30) Priorité: 29.06.2015 FR 1556027
(43) Date de publication de la demande: 02.05.2018
(73) Titulaire: One Ortho, 69230 Saint-Genis-Laval (FR)
(72) Inventeur: ALEPEE, Christophe, 69003 Lyon (FR); GUITON, Thierry, 76100 Rouen (FR)
(74) Mandataire: Cabinet Laurent & Charras
(86) Numéro de dépôt international: PCT/FR2016/051535
(87) Numéro de publication internationale: WO 2017/001748

(56) Documents cités:
- EP-A1- 2 510 896
- US-A1- 2012 109 137
- US-A1- 2012 116 203
- US-A1- 2015 057 702

## Description

### DOMAINE TECHNIQUE

La présente invention se rapporte au secteur technique de la chirurgie, telle que la chirurgie orthopédique, et concerne plus particulièrement un procédé de fabrication d'une instrumentation jetable constituée d'au moins deux pièces, standards ou conçues sur mesure pour un patient pour la réalisation d'une opération chirurgicale.

L'invention concerne également l'ensemble d'instruments jetables en tant que tel.

L'état de la technique le plus proche est donné par le document EP 2510896 A1, qui définie les préambules des revendications indépendantes.

### ETAT ANTERIEUR DE LA TECHNIQUE

Dans la chirurgie, par exemple orthopédique, il est connu de réaliser des instrumentations jetables, par exemple adaptées à un patient en particulier. Par instrumentation on entend tout instrument nécessaire à la réalisation d'une opération chirurgicale, par exemple pour la pose et la mise en place de différents implants orthopédiques, telle qu'une prothèse de genou.

Les pièces constitutives de l'ensemble d'instruments sont, par exemple, des implants d'essais, et des instruments de pose tels que des guides de perçage, des préhenseurs ou des impacteurs. Lesdites pièces sont réalisées en matière plastique par une technique de fabrication additive, telle que par une impression 3D.

Par jetable, on entend à usage unique, pour une seule opération chirurgicale.

La fabrication dite additive consiste à fabriquer, couche par couche, les pièces constitutives de l'instrumentation. Cette technique de fabrication permet la réalisation d'un très grand nombre de pièces, par exemple sur mesure, pour de nombreux patients, dans un volume de production réduit.

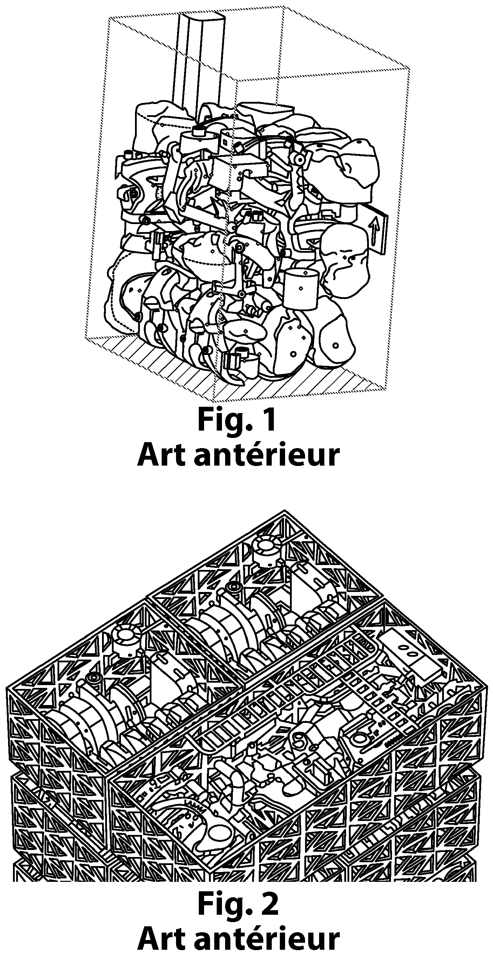

La figure 1 représente, à titre d'exemple, un volume de production d'une pluralité de pièces adaptées à des patients distincts. Ce volume de production est également connu sous le nom de « charge ».

Cependant, un premier inconvénient lié à cette technique de fabrication d'une pluralité de pièces destinées à des patients différents, dans un volume de production réduit, réside dans la traçabilité des pièces d'un patient, ainsi que dans le risque de perte et ou de mélange de deux pièces de deux patients ou de deux lots d'instruments de tailles différentes.

En effet, la production d'une instrumentation jetable avec cette technologie permet de produire entre 1 et 50 pièces pour un même patient et, sachant que des pièces sont produites pour plusieurs patients en même temps, il fortement possible de mélanger les pièces de deux patients ou de deux lots d'instruments différents. Lesdites pièces peuvent, certes, être marquées par une référence spécifique au patient concerné, mais le fait que ces pièces soient blanches n'aident pas à la lecture de ces références et complique ainsi la traçabilité.

Pour pallier à ce premier inconvénient lié à la traçabilité des pièces de chaque patient, il a déjà été envisagé de fabriquer par une technique de fabrication additive et en même temps que les pièces, des boîtes en plastiques renfermant ensemble toutes les pièces d'un même patient, telles qu'illustrées à la figure 2, communément appelées « Sinter Box » par les fabricants de machine.

Cette technique permet de ne pas mélanger les pièces pendant l'étape de production, mais également de ne pas perdre des pièces de petites dimensions. Cependant, après l'étape de production, il convient de nettoyer et de traiter lesdites pièces, par sablage ou projection de microbilles pour enlever des résidus de poudre de plastique issus de l'opération de fabrication. Pour effectuer ce traitement, il convient d'ouvrir les boîtes en plastique pour retirer les pièces et les traiter. Le risque de perdre ou de mélanger les pièces de deux patients ou de deux lots différents sont de nouveau présents à ce moment-là. Cette solution n'assure donc pas une parfaite traçabilité des pièces jusqu'à l'opération chirurgicale sur le patient, et ne supprime pas entièrement le risque de perdre ou de mélanger deux pièces de deux patients ou de deux lots différents.

La fabrication additive consiste à déposer sur une table une couche de poudre de plastique, de quelques centièmes de centimètres d'épaisseur, et de fusionner une section utile de cette couche au moyen d'un faisceau laser. La table descend ensuite de quelques centièmes de centimètres et une nouvelle couche de poudre est déposée puis une section utile de cette couche est fusionnée. Ainsi, la pièce est réalisée couche par couche, lesquelles couches sont fusionnées par un faisceau laser et additionnées les unes aux autres.

De cette manière, lorsqu'une couche supérieure de poudre est fusionnée sur une couche inférieure préalablement fusionnée, la qualité de la couche supérieure fusionnée n'est pas dégradée. Cependant, lorsqu'une couche supérieure est fusionnée et qu'elle repose sur une couche inférieure de poudre non fusionnée, il se produit un effet de dégradation dimensionnelle et morphologique de la qualité de la couche supérieure fusionnée.

En effet, lorsqu'une couche supérieure de poudre est déposée sur une couche inférieure de poudre non fusionnée, la couche supérieure de poudre à tendance à se loger dans les interstices de la couche inférieure de poudre de sorte qu'après fusion de la couche supérieure, celle-ci ne présente pas une qualité optimale, notamment aux niveaux de ses bords qui s'arrondissent ce qui peut poser des problèmes de dimensionnement.

Ceci entraine un second inconvénient lorsqu'il convient de réaliser des pièces sur mesure avec une extrême précision.

### EXPOSE DE L'INVENTION

L'un des buts de l'invention est donc de remédier aux inconvénients précités en proposant un procédé de fabrication d'un ensemble d'instruments jetables, standards ou conçus sur mesure pour une opération chirurgicale d'un patient, qui permette d'assurer une traçabilité desdits instruments jusqu'à l'opération chirurgicale sur le patient. En d'autres termes, le procédé de fabrication selon l'invention vise à éviter de perdre des pièces constitutives de l'ensemble d'instruments ou de les mélanger avec celles d'un autre patient ou d'un autre ensemble d'instruments de tailles différentes.

Un autre objectif de l'invention est de fournir un procédé qui permette de fabriquer des pièces avec un positionnement maitrisé pour assurer un bon dimensionnement desdites pièces.

Un autre objectif de la présente invention vise à garantir un marquage plus lisible pour référencer et identifier les pièces propres à un patient.

A cet effet, le procédé de fabrication consiste à fabriquer, en même temps et par fabrication additive couche par couche, des pièces constitutives de l'ensemble d'instruments ainsi que des pattes de liaison, liées de manière sécable aux pièces, et reliant entre elles lesdites pièces pour former ledit ensemble.

De cette manière, l'ensemble d'instruments ne forme qu'un seul ensemble comprenant au moins deux pièces, standards ou conçues sur mesure pour un patient, reliées entre elles par des pattes de liaison permettant d'éviter de perdre les pièces ou de mélanger deux pièces de deux patients ou de deux ensembles d'instruments de tailles différentes. Après fabrication, les pièces sont traitées et nettoyées ensemble, avec leurs pattes de liaison. Ce n'est que lors de l'opération chirurgicale que les pattes, dont la liaison avec les pièces est sécable, sont cassées par le chirurgien pour détacher une à une lesdites pièces.

L'invention permet également de maitriser le positionnement et l'orientation des pièces à fabriquer par rapport à la succession des couches produites, ce qui est très important pour assurer une qualité optimale des parties de pièces nécessitant une précision extrême et assurer le bon dimensionnement desdites pièces.

L'invention concerne également un ensemble d'instruments jetables standards ou conçus sur mesure pour une opération chirurgicale d'un patient. Selon l'invention, l'ensemble d'instruments jetables comprend au moins deux pièces reliées entre elles par au moins une patte de liaison liée de manière sécable auxdites pièces.

De préférence, la ou les pattes de liaison sont planes et pourvues d'une inscription, de préférence traversante, identifiant les références des pièces et/ou le patient auxquelles lesdites pièces sont adaptées.

Avantageusement, les pièces sont reliées entre elles dans l'ordre chronologique dans lequel elles doivent être utilisées au cours de l'opération chirurgicale.

Selon une forme de réalisation particulière, les pièces comprennent au moins un orifice, de préférence de forme tronconique ou hémisphérique s'évasant en direction de la surface externe de la pièce, le fond dudit orifice étant lié de manière sécable par une zone fragilisée avec une extrémité en pointe d'une patte de liaison.

De cette manière, les pièces sont attachées aux pattes de liaison de manière fiable et forment en quelque sorte une « grappe » de pièces chirurgicales. De plus, la liaison entre les pattes de liaison et les pièces est sécable et évite, après détachement, de former des pointes de plastique en surface des pièces qui pourraient déchirer les gants du chirurgien. En effet, après séparation des pièces et des pattes de liaison, les pointes éventuellement formées par le fait de casser lesdites pattes de liaison sont cachées à l'intérieur des orifices desdites pièces.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées dans lesquelles :
- la figure 1 est une vue schématique en perspective illustrant un volume de production, conformément à l'art antérieur, d'une pluralité de pièces adaptées à des patients distincts ;
- la figure 2 est une vue schématique en perspective illustrant la technique de fabrication de l'art antérieur consistant à fabriquer des boîtes en plastiques renfermant ensemble toutes les pièces d'un même patient ;
- la figure 3 est une vue schématique illustrant de face un ensemble d'instruments jetables selon l'invention ;
- la figure 4 est une vue schématique similaire à celle de la figure 3, l'ensemble d'instruments jetables étant illustré en coupe longitudinale ;
- la figure 5 est une vue de détail en coupe longitudinale illustrant la liaison sécable entre une extrémité en pointe d'une patte de liaison et une pièce chirurgicale
- la figure 6 est une vue schématique similaire à celle de la figure 3, l'ensemble d'instruments jetables étant illustré vue de dessus.

### EXPOSE DETAILLE DE L'INVENTION

L'invention concerne un procédé de fabrication d'instruments chirurgicaux en plastiques, standards, ou conçus sur mesure pour une opération chirurgicale d'un patient, par exemple comprenant des pièces d'une même taille, tels que des implants d'essais, des instruments de pose, tels que des guides de coupe ou de perçage, des préhenseurs, ou des impacteurs.

L'invention consiste à fabriquer les pièces constitutives de l'ensemble d'instruments par fabrication additive, couche par couche, par exemple au moyen d'une imprimante 3D. Les pièces obtenues sont à usage unique et ont un prix de revient relativement faible.

Les pièces sont fabriquées couche par couche, en même temps avec des pattes de liaison, liées de manière sécable auxdites pièces, et agencées de sorte à relier entre elles lesdites pièces pour former un seul ensemble de pièces adaptées à un seul patient ou un seul ensemble de pièces d'une même taille. Cette technique permet d'éviter de perdre des pièces et/ou de les mélanger avec celles d'un autre patient ou d'un autre lot d'instruments de tailles différentes.

Ces pièces, adaptées à un patient spécifique, restent solidaires les unes des autres pendant l'étape de nettoyage et de traitement qui suit l'étape de fabrication, et notamment jusqu'à leur utilisation en bloc opératoire. Les pièces d'un même ensemble sont destinées à être séparées des pattes de liaison par le chirurgien lui-même. De préférence, les pièces sont reliées entre elles dans l'ordre chronologique dans lequel elles doivent être utilisées au cours de l'opération chirurgicale, et de préférence également orientées de façon à assurer un dimensionnement optimal desdites pièces.

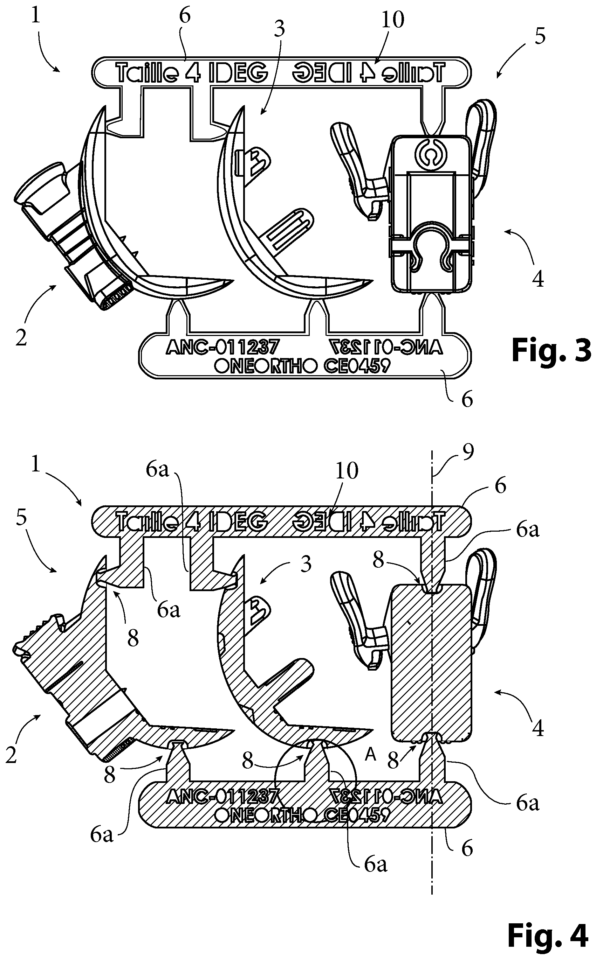

En référence aux figures 3 à 6, l'ensemble d'instruments jetables, référencé (1) fabriqué selon l'invention comprend trois pièces (5) pour la pose d'une prothèse de genou, dont un guide de perçage (2), un implant d'essai (3) et un préhenseur (4) pour impacteur. Lesdites pièces (5) sont alignées et reliées entre elles par deux pattes de liaison (6) disposées de part et d'autre du jeu de pièces (5). Chaque pièce (5) est reliée aux deux pattes de liaison (6) par des liaisons sécables (7). A cet effet, chaque pièce (5) comprend deux orifices (8) de forme tronconique ou hémisphérique s'évasant en direction de la surface externe de la pièce (5), chacun ménagé sur la pièce (5) à proximité d'une patte de liaison (6).

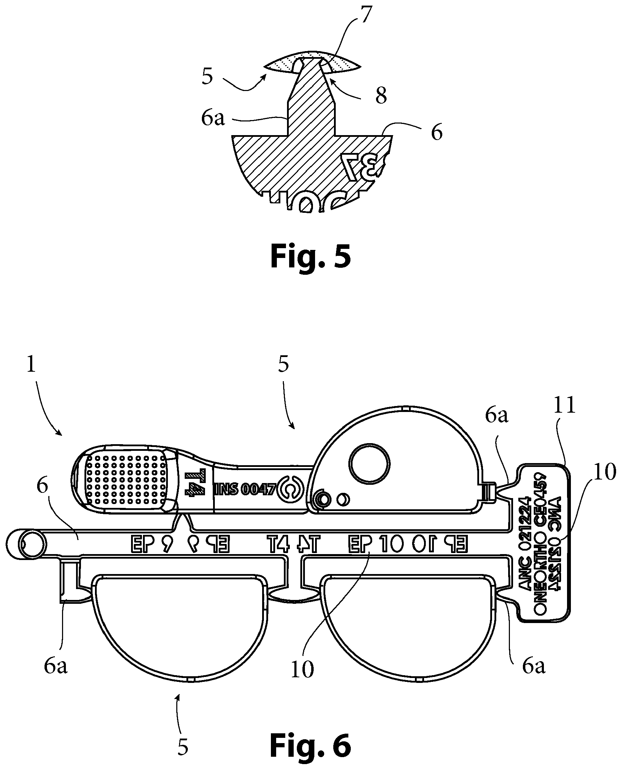

Plus précisément, et en référence à la figure 5, le fond de chaque orifice (8) est lié de manière sécable par une zone fragilisée, notamment de faibles dimensions, avec une extrémité en pointe d'une patte de liaison (6). Dans l'exemple illustré, chaque patte de liaison (6) comprend trois parties (6a) s'étendant en direction des pièces (5) dont les extrémités sont en pointes et sont liées de manière sécable à l'intérieur des orifices (8).

De préférence, les orifices (8) ménagés sur une même pièce (5) sont alignés le long d'une même génératrice (9) pour permettre de faciliter le détachement de ladite pièce (5). En effet, pour détacher ladite pièce (5), il suffit de la faire tourner autour de l'axe de la génératrice (9).

La traçabilité est donc améliorée car il est dorénavant possible de suivre les pièces (5) d'un même patient dès leur fabrication jusqu'à leur utilisation. Pour faciliter davantage la traçabilité desdites pièces (5), la ou les pattes de liaison (6) comprennent des inscriptions (10) identifiant les références des pièces (5) et/ou le patient auquel elles sont destinées. Les pattes de liaison (6) sont avantageusement planes pour faciliter l'inscription et lesdites inscriptions (10) traversent de préférence l'épaisseur de la patte de liaison (6) pour faciliter la lecture desdites inscriptions (10). En référence à la figure 6, l'invention permet également de prévoir lors de la fabrication des zones (11) dites « étiquettes » permettant de recevoir des inscriptions (10) d'identification supplémentaires.

L'invention permet alors de relier entre elles les pièces (5) d'un même patient. Il est également possible de contrôler et de maitriser le positionnement et l'orientation des pièces (5) dans l'ensemble (1) selon l'invention. En effet, comme évoqué, les pièces (5) peuvent être agencées dans l'ordre chronologique dans lequel elles doivent être utilisées lors de l'opération chirurgicale, mais elles peuvent également être orientées de sorte à positionner les faces ne nécessitant pas une très grande précision, de manière orthogonale par rapport au faisceau laser incident pour la fusion. Ainsi, le dimensionnement des pièces (5) est optimal et l'on peut assurer une précision extrême desdites pièces (5).

Comme il ressort de ce qui précède, l'invention fournit un procédé de fabrication d'un ensemble (1) d'instruments jetables et l'ensemble (1) d'instruments en tant que tel, standard ou conçu sur mesure pour une opération chirurgicale d'un patient, qui permettent d'assurer une traçabilité desdits instruments jusqu'à l'opération chirurgicale sur le patient, et évitant de perdre des pièces (5) constitutives de l'ensemble (1) d'instruments ou de les mélanger avec celles d'un autre patient ou d'un autre ensemble d'instruments de tailles différentes, tout en maitrisant l'orientation des pièces (5) pour leur assurer un bon dimensionnement. L'invention garantie également un marquage plus lisible pour référencer et identifier les pièces (5) propres à un patient.

## Revendications

1. Procédé de fabrication d'un ensemble (1) d'instruments jetables pour une opération chirurgicale d'un patient, ***caractérisé* en ce qu'**il consiste à fabriquer, en même temps et par fabrication additive couche par couche, des pièces (5) constitutives de l'ensemble (1) d'instruments ainsi que des pattes de liaison (6), liées de manière sécable aux pièces (5), et reliant entre elles lesdites pièces (5) pour former ledit ensemble (1).

2. Ensemble (1) d'instruments jetables pour une opération chirurgicale d'un patient, ***caractérisé* en ce qu'**il comprend au moins deux pièces (5) reliées entre elles par au moins une patte de liaison (6) liée de manière sécable auxdites pièces (5).

3. Ensemble (1) d'instruments jetables selon la revendication 2, ***caractérisé* en ce que** la ou les pattes de liaison (6) sont planes et pourvues d'une inscription (10) identifiant les références des pièces (5) et/ou le patient auxquelles lesdites pièces (5) sont adaptées.

4. Ensemble (1) d'instruments jetables selon la revendication 3, ***caractérisé* en ce que** l'inscription (10) traverse l'épaisseur de la patte de liaison (6).

5. Ensemble (1) d'instruments jetables selon l'une quelconque des revendications 2 à 4, ***caractérisé* en ce que** les pièces (5) sont reliées entre elles dans l'ordre chronologique dans lequel elles doivent être utilisées au cours de l'opération chirurgicale.

6. Ensemble (1) d'instruments jetables selon l'une quelconque des revendications 2 à 4, ***caractérisé* en ce que** les pièces (5) comprennent au moins un orifice (8) dont le fond est lié de manière sécable, par une zone fragilisée, avec une extrémité en pointe d'une patte de liaison (6).

7. Ensemble (1) d'instruments jetables selon la revendication 6, ***caractérisé* en ce que** les orifices (8) sont de forme tronconique ou hémisphérique en s'évasant en direction de la surface externe de la pièce (5).

## Patentansprüche

1. Verfahren zur Herstellung eines Satzes (1) Einweginstrumente für eine chirurgische Operation eines Patienten, ***dadurch gekennzeichnet,* dass** es darin besteht, gleichzeitig und durch zusätzliche Herstellung, schichtweise, die Teile (5) herzustellen, die den Instrumentensatz (1), sowie Verbindungslaschen (6) bilden, die abtrennbar mit den Teilen (5) verbunden sind und diese Teile (5) miteinander verbinden, um diesen Satz zu bilden (1).

2. Satz (1) Einweginstrumente, für eine chirurgische Operation eines Patienten, ***dadurch gekennzeichnet,* dass** er mindestens zwei miteinander verbundene Teile (5) enthält, die miteinander über eine Verbindungslasche (6) verbunden sind, die mit diesen Teilen (5) abtrennbar verbunden ist.

3. Satz (1) Einweginstrumente nach Anspruch 2, ***dadurch gekennzeichnet,* dass** die eine oder mehreren Verbindungslaschen (6) flach und mit einer Aufschrift (10) zur Identifikation der Referenzen der Teile (5) und/ oder des Patienten, für den diese Teile (5) geeignet sind, versehen sind.

4. Satz (1) Einweginstrumente nach Anspruch 3, ***dadurch gekennzeichnet*, dass** die Aufschrift (10) durch die Dicke der Verbindungslasche (6) hindurchführt.

5. Satz (1) Einweginstrumente, nach einem der vorstehenden Ansprüche 2 bis 4, ***dadurch gekennzeichnet*, dass** die Teile (5) miteinander in der chronologischen Reihenfolge verbunden sind, in dem sie während des chirurgischen Eingriffs eingesetzt werden müssen.

6. Satz (1) Einweginstrumente, nach einem der vorstehenden Ansprüche 2 bis 4, ***dadurch gekennzeichnet*, dass** die Teile (5) mindestens eine Öffnung (8) umfassen, deren Boden in abtrennbarer Weise über eine geschwächte Zone mit einem spitzen Endstück einer Verbindungslasche (6) verbunden ist.

7. Satz (1) Einweginstrumente nach Anspruch 6, ***dadurch gekennzeichnet*, dass** die Öffnungen (8) kegelstumpfförmig oder halbkugelförmig sind und sich in Richtung der Außenfläche des Teils (5) erweitern.

## Claims

1. A method for producing a set (1) of disposable instruments for a surgical procedure on a patient, ***characterized* in that** it consists of making, at the same time and by additive production layer by layer, components (5) forming the set (1) of instruments and connecting tabs (6), connected separably to the components (5), and connecting said components together (5) to form said set (1).

2. A set (1) of disposable instruments for a surgical procedure on a patient, ***characterized* in that** it comprises at least two components (5) connected by at least one connecting tab (6) connected separably to said components (5).

3. The set (1) of disposable instruments according to claim 2, ***characterized* in that** the connecting tab or tabs (6) are flat and provided with an inscription (10) identifying the references of the components (5) and/or the patient for whom said components (5) are tailored.

4. The set (1) of disposable instruments according to claim 3, ***characterized* in that** the inscription (10) crosses through the thickness of the connecting tab (6).

5. The set (1) of disposable instruments according to any one of claims 2 to 4, ***characterized* in that** the components (5) are connected in the chronological order in which they must be used during the surgical procedure.

6. The set (1) of disposable instruments according to any one of claims 2 to 4, ***characterized* in that** the components (5) comprise at least one orifice (8) whose base is connected separably, by a weakened area, with a pointed end of a connecting tab (6).

7. The set (1) of disposable instruments according to claim 6, ***characterized* in that** the orifices (8) are shaped as a truncated cone or hemisphere and widen towards the external surface of the component (5).
